(19) Europäisches Patentamt
European Patent Office
Office européen des brevets

(11) Publication number: **0 084 691 A1**

# (12) EUROPEAN PATENT APPLICATION

(21) Application number: **82201611.9**

(51) Int. Cl.³: **C 07 C 103/52**

(22) Date of filing: **16.12.82**

(30) Priority: **22.12.81 IT 2575581**

(43) Date of publication of application: **03.08.83 Bulletin 83/31**

(84) Designated Contracting States: **AT BE CH DE FR GB LI LU NL SE**

(71) Applicant: **ANIC S.p.A., Via Ruggero Settimo, 55, I-90139 Palermo (IT)**

(72) Inventor: **Verdini, Antonio Silvio, Via S. Martino 12, I-00015 Monterotondo (Rome) (IT)**
Inventor: **Viscomi, Giuseppe Claudio, Via 8 Maggio 23, I-00015 Monterotondo(Rome) (IT)**

(74) Representative: **Roggero, Sergio et al, Ing. Barzanò & Zanardo Milano S.p.A. Via Borgonuovo 10, I-20121 Milano (IT)**

## (54) Method for preparing n-monoacylated gem-diamino derivatives.

(57) This invention relates to a new method for preparing N-monoacylated gem-diamino derivatives of general formula I from an aminoacid, hydroxyacid, peptide or depsipeptide residue with the terminal $-NH_2$ or $-OH$ groups protected

$$P-(A)_n-NH-\underset{\displaystyle R}{\underset{|}{CH}}-NH_3{}^+X^- \qquad (I)$$

In particular, the method according to the present invention consists of directly converting primary linear aminoacid, hydroxyacid, peptide or depsipeptide amides protected at the terminal $-NH_2$ or $-OH$, of general formula II

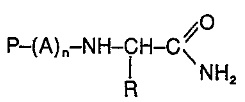

(II)

into the corresponding primary amines of general formula I by reacting with I,I-bis(trifluoroacetoxy)iodobenzene.

The derivatives of general formula I are important intermediates in the synthesis of biologically active retro-inverso peptides which are stable to enzymatic degradation.

EP 0 084 691 A1

CASE 1463

0084691

"Method for preparing N-monoacylated gem-diamino derivatives"

Inversion of the direction of the amide bonds of peptide chains (from 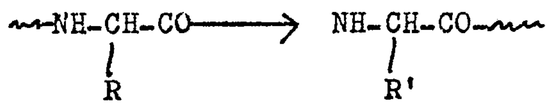 to

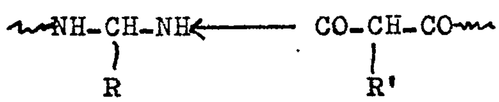 ) enables biologically active peptide analogues to be obtained which besides being an interesting object of study in that they make it possible to determine the relative importance of the side chains and peptide skeleton in biological activity, can find useful practical application in pharmacology because of their high stability to enzymatic degradation (Goodman M. and Chorev M, Acc. Chem. Res. 12. 1 (1979) and cited references). This modification of the peptide skeleton accompanied when necessary by inversion of the chirality of some of the aminoacid residues in the chain and by the modification of the terminal groups is carried out with the intention of maintaining the topology of the side chains unaltered.

The resultant analogues, known generally as retro-inverso peptides, are structural isomers which although not exactly identical with the natural reference peptides from the topochemical aspect, can retain their biological activity. In particular, in the case of cyclic peptides and depsipeptides, it has been shown that inversion of the direction of the peptide skeleton accompanied by inversion of each of the chiral centres of the chain produces structural isomers with complete biological activity (Shemyakin M.M., Ovchinnikov,

Y.A. and Ivanov, V.T., Angew. Chem. 8, 492 (1969)). It is apparent that in these compounds the biological activity depends on the three-dimensional orientation of the side chains rather than on the structure of the peptide skeleton. In the case of retro-inverso analogues of linear peptides with all the peptide bonds inverted, the existence of the terminal groups poses particular problems for maintaining the steric complementarity with the reference peptide. In general, and in the case of linear reference peptides with amino chain terminals and free carboxyls, the problem is solved by transforming the N-terminal aminoacid residue into a gem-diamino residue, transforming the C-terminal aminoacid residue into a malonyl residue or a malonyl residue substituted in position 2, and inserting the intermediate residues in the chain with inverted configuration.

If not all the peptide bonds of the chain are retro-inverted, partially modified retro-inverso analogues are obtained in which the modified chain segments are incorporated between normal segments.

The inversion of a single peptide bond produces two adjacent non-aminoacid residues in which the residue closer to the amino end is of gem-diamino type, whereas that closer to the carboxyl end is of malonyl or 2-substituted malonyl type. The extension of the modification to non-adjacent peptide bonds leads to inversion of the chirality of the aminoacids interposed between the two non-aminoacid residues.

Up to the present time, biologically active retro-inverso

analogues of linear peptides have been synthesised such as angiotensin, bradykinin, luteotropic hormone releasing hormone, thyrotropin releasing hormone, encephalin, tuftsin, C-terminal tetrapeptide of desamino gastrin, 5-9 pentapeptide of α-melanotropin, Substance $P_{5-11}$ and aspartame (Goissis G. et al., J. Med. Chem. 19, 1287 (1976); Vogler K., et al., Helv. Chim. Acta, 49, 390 (1966); Chorev M. et al. in "Peptides: Proceedings of the 5th American Peptide Symposium", Goodman M. and Meienhofer J., Editors, Wiley, New York 1977, p. 572; Goodman M. and Chorev M. in "Perspectives in Peptide Chemistry", Eberle A., Geiger R. and Wieland T., Editors, Karger, Basel, 1980, p. 283; Chorev M. et al., Science 204, 1210 (1979); Hayward C.F. and Morley J.S. in "Peptides 1974, Proceedings of the 13th European Peptide Symposium", Volman Y. Ed., Wiley, New York and Israel Universities Press, Jerusalem, 1975, p. 287; Chung D. and Li C.H., Biochim. Biophys. Acta 136, 570 (1967); Cuignet E. et al. in "Peptides 1980, Proceedings of the 16th European Peptide Symposium", Brunfeldt K. Ed., Scriptor, Copenhagen, 1981, p. 608).

In particular, the synthesis of four analogues of encephalinamide with inversion of the peptide bond between the four and five residues, with or without simultaneous inversion of the terminal carboxyamide bond, has produced new and powerful topochemical analogues of the encephalins.

In this case, the inversion of part of the amide bonds of the chain has enabled not only the biological activity to be preserved unaltered but also to potentiate it, probably by

the effect of the increased resistance to enzymatic hydrolysis in vivo.

While the incorporation of the malonyl or 2-substituted malonyl residues into the skeleton of these peptides has not presented particular problems, that of the gem-diaminoalkyl residues has required special and delicate synthesis manipulations. The sequence of reactions used can be summarised as follows: an aminoacid or peptide azide suitably protected at the $NH_2$ is firstly converted into the corresponding isocyanate by means of Curtius rearrangement. The isocyanate intermediate is then subsequently utilised either in reactions with the carboxyl component to give the mixed carbamic-carboxylic anhydride, which by thermal elimination of carbon dioxide produces the desired amide bond, or is captured by reaction with an excess of an appropriate alcohol (in general tertiary butyl alcohol or benzyl alcohol) to give a new type of urethan protector group for the gem-diamino residue.

This new amino protector group is eliminated immediately before the condensation reaction with the carboxyl component because of the instability of the gem-diamino alkyl derivatives (Goodman M. and Chorev M., Acc. Chem. Res., 12, 1 (1979)).

The isocyanate intermediates are prepared in their turn by three different synthesis procedures: a) reacting the carboxyl component with diphenylphosphorylamide in toluene, followed by heating in the presence of triethylamine (Wilson C.G. et al., in "Peptides: Proceedings of the 5th American Peptide Symposium", Goodman M. and Mienhofer J., Editors,

Wiley, New York, 1977, p. 579); b) reacting the mixed carbamic-carboxylic anhydride under cold conditions with an excess of sodium azide in water to give the corresponding acyl azide, followed by thermal rearrangement (Sheehan, J.C. et al., J. Org. Chem. 42, 4045 (1977)); c) reacting an acyl hydrazide with nitrosyl chloride in anhydrous tetrahydrofuran to give the corresponding azide, followed by thermal rearrangement (Honzl - Rudinger process) (Honzl J. and Rudinger J., Coll. Czech. Chem. Commun. 26, 2333 (1961)).

We have now devised a method, constituting the subject matter of the present invention, which makes it possible to introduce a gem-diamino residue into a peptide skeleton in a particularly easy manner without special tedious chemical manipulations, by using I,I-bis(trifluoroacetoxy)iodobenzene. This new reagent has been used recently for the direct conversion of primary carboxyl amides of simple structure into amines under extremely mild reaction conditions without the need to isolate or capture the intermediate isocyanate (Radhakrishna A.S. et al., J. Org. Chem. 44, 1746 (1979)).

We have now found that said procedure can be suitably adapted to the direct conversion of primary aminoacid, hydroxyacid, peptide or depsipeptide amides protected at the terminal $-NH_2$ or -OH, of general formula II, into the corresponding trifluoroacetic acid salts of N-monoacylated gem-diamino derivatives of general formula I, in accordance with the following scheme:

$$P\text{-}(A)_n\text{-}NH\text{-}CH\text{-}CO\text{-}NH_2 \; + \; C_6H_5I(OCOCF_3)_2$$

$$\xrightarrow[-CO_2]{H_2O/\text{organic solvent}}$$

$$P\text{-}(A)_n\text{-}NH\text{-}\underset{\displaystyle R}{\underset{|}{CH}}\text{-}NH_3^+ \; CF_3COO^- + CF_3COOH + C_6H_5I$$

(I)

In said formulas P is a benzyloxycarbonyl group (Z), a tert-butyloxycarbonyl group (Boc), an acyl residue or generally any $NH_2$ or OH protector group stable under reaction conditions; A is a residue of an aminoacid or hydroxyacid which can be either natural (such as valene, alanine, tyrosine, methionine, lactic acid, mandelic acid etc.) or synthetic (4-methyl-proline, trifluoroalanine etc.); R is a hydrogen atom, or an alkyl, phenyl, phenylalkylene, hydroxyphenylalkylene, hydroxy-alkylene, aminoalkylene, acylaminoalkylene, guanidinylalkylene, imidazolylalkylene, indolylalkylene, mercaptoalkylene, alkylmercaptoalkylene or carboxyalkylene group or their suitable derivatives; n is either 0, 1 or greater than 1, in which case the aminoacid sequence represented by $(A)_n$ can be constituted by residues which are the same or different and, if different, disposed at random or in predetermined sequence; or the sequence represented by $(A)_n$ can be constituted by aminoacid residues which are the same or different, or hydroxyacid residues which are the same or different, either disposed at random or in a predetermined sequence.

The described invention is generally applicable to amides of general formula II, with yields which normally are of the

order of 80-90%.

Pilot reactions are used as required in order to determine the optimum reaction conditions, in particular with regard to the duration of the reaction and the molar excess of reagent. The reaction, carried out under effective agitation of the initial solution or suspension and bubbling inert gas through the reaction mixture, requires from two to six hours to attain completion.

On termination of the reaction, the mixture is evaporated to dryness, the residue is taken up in a suitable organic solvent and treated with a stoichiometric quantity of HCl dissolved in an organic solvent miscible with the former, to obtain, either directly or by adding a suitable quantity of ethyl ether, the hydrochloride of the gem-diamino derivative protected at the terminal $-NH_2$ or -OH in crystalline form.

The final products, prepared in accordance with the described methods, can be isolated in their pure state, by crystallising the hydrochlorides in the case of the shorter peptides and depsipeptides (2-5 residues), or by chromatography separation in the case of more complex peptides. The hydrochlorides of the gem-diamino derivatives protected at the terminal $-NH_2$ or -OH can be preserved without appreciable degradation in an inert gas atmosphere under cold conditions.

Two examples are reported hereinafter involving the use of gem-diamino derivatives protected at the terminal $NH_2$ in condensation reactions with carboxyl components constituted by aminoacid or peptide derivatives. The carboxyl which

participates in the condensation can pertain to aminoacids or malonyl or 2-substituted malonyl residues, either free or bonded to peptide fragments. For example, products of the condensation of gem-diamino derivatives with the said carboxyl components induced by dicyclohexylcarbodiimide and catalysed by N-hydroxybenzotriazole are generally isolated with high yields by the known standard procedures of peptide synthesis. A characteristic of fundalmental importance of this invention is that the described process can be applied with substantially total control of racemisation.

In this respect, we have verified, by appropriate synthesis and the application of high pressure liquid chromatography, that the transformation of the peptide amides Boc-Phe-Phe-$NH_2$ and Boc-Phe-D Phe-$NH_2$ into the corresponding analogues Boc-Phe-g Phe-H.HCl and Boc-Phe-g D Phe-H.HCl (where g Phe represents the corresponding gem-diamino Phe derivative) takes place with substantial configuration retention (degree of racemisation undetectable by the analytical methods available).

The subject matter and scope of the invention will be more apparent from reading the following examples, which are simply illustrative and must in no manner be considered limitative of the invention.

The product purity was demonstrated by reverse phase high pressure chromatography analysis (RP-HPLC) using the following eluent systems: $H_2O$/acetonitrile; 0.01 M $NH_4H_2PO_4$/acetonitrile; 0.005 M heptanesulphonic acid-0.01 M $NH_4H_2PO_4$/acetonitrile. and chromatography analysis on a thin layer of silica gel using the

following eluent systems: n-butanol-acetic acid-water (4/1/1); chloroform-methanol-acetic acid (85:10:5); n-butanol-isopropanol-1N $NH_4OH$-ethyl acetate (1:1:5:1) organic phase. The melting points have not been corrected. The configurations of the aminoacids and derivatives are L where not specified.

EXAMPLE 1

Synthesis of tert-butyloxycarbonyl-L-phenylalanyl-gem-diamino-L-phenylalanine hydrochloride. Boc-Phe-g Phe-H.HCl

1 equivalent of Boc-Phe-Phe-$NH_2$ is suspended in a 3:2 (v/v) mixture of acetonitrile-water, and 1.2 equivalents of I,I-bis (trifluoroacetoxy)iodobenzene (BTI) dissolved in acetonitrile are added to the suspension at ambient temperature under vigorous agitation.

An inert gas is bubbled through the reaction mixture in order to facilitate the removal of the carbon dioxide which develops during the course of the reaction. Having verified the disappearance of Boc-Phe-Phe-$NH_2$, the reaction is suspended about five hours after adding the reagent by evaporating to dryness, the residue is washed with ethyl ether, dried and dissolved in ethanol. A stoichiometric quantity of HCl dissolved in ethyl acetate is added to this solution in order to induce the complete precipitation of Boc-Phe-g Phe-H.HCl during the course of two hours. The precipitate is filtered, washed abundantly with various portions of ethyl ether, dried under vacuum in $P_2O_5$ and collected. M.P. = 174°C (dec.).

$[\alpha]^{546}_{20} = -48.8°$ (C = 1 in dimethylformamide).

Elementary analysis for $C_{22}H_{30}O_3N_3Cl$:

Theoretical: C, 62.94%; H, 7.15%; N, 10.01%

Found: C, 62.39%; H, 7.12%; N, 10.27%.

Chromatography analysis (t.l.c. and HPLC) shows no presence of impurities, and the $^{1}H$ n.m.r. spectrum confirms the molecular structure.

EXAMPLE 2

Synthesis of tert-butyloxycarbonyl-L-phenylalanyl-gem-diamino-L-phenylalanine hydrochloride. Boc-Phe-g-D-Phe-H.HCl

1 equivalent of Boc-Phe-D-Phe-$NH_2$ is dissolved in a 3:2 (v/v) mixture of acetonitrile-water, and 1.2 equivalents of BTI dissolved in acetonitrile are added to the solution at ambient temperature under energetic agitation. An inert gas is bubbled through the solution to facilitate removal of the carbon dioxide which develops during the reaction. Having verified the disappearance of the starting amide after four hours, the reaction is suspended by evaporating the solvent mixture to dryness, the residue is washed with ethyl ether, dried and dissolved in ethanol. A stoichiometric quantity of HCl dissolved in ethyl acetate is added to this solution, after which ethyl ether is suitably added under cold conditions to give the required product in crystalline form. M.P. = 131-133°C (dec.).

$[\alpha]^{546}_{20}$ = 52.0 (C = 1 in dimethylformamide).

Elementary analysis for $C_{22}H_{30}O_3N_3Cl$:

Theoretical: C, 62.94%; H, 7.15%; N, 10.01%

Found: C, 62.58%; H, 7.00%; N, 9.91%.

Chromatography analysis (t.l.c. and HPLC) shows no presence of impurities, and the $^{1}H$ n.m.r. spectrum confirms the molecular structure.

EXAMPLE 3

Synthesis of tert-butyloxycarbonyl-L-alanyl-gem-diamino-L-phenylalanine hydrochloride. Boc-Ala-g Phe-H.HCl

1 equivalent of Boc-Ala-Phe-$NH_2$ is dissolved in a 3:2 (v/v) mixture of acetonitrile-water, and 1.2 equivalents of BTI dissolved in acetonitrile are added to the solution at ambient temperature under agitation. During the reaction, which lasts for about six hours, an inert gas is bubbled into the reaction vessel to favour the elimination of the developed carbon dioxide. Having verified the disappearance of Boc-Ala-Phe-$NH_2$, the reaction solvent is evaporated, the residue dissolved in ethyl acetate and treated with a stoichiometric quantity of HCl dissolved in ethyl acetate. The resultant precipitate is filtered, washed abundantly with ethyl ether and dried under vacuum in the presence of $P_2O_5$. Any traces of impurities can be eliminated by dissolving the product in ethanol, adding ethyl ether and maintaining it at about 4°C until complete crystallisation takes place. M.P. = 137°C (dec.).

$[\alpha]^{546}_{20}$ = 37.0 (C = 1.0 in dimethylformamide).

Elementary analysis for $C_{16}H_{26}N_3O_3Cl$:

Theoretical: C, 55.90%; H, 7.57%; N, 12.23%

Found: C, 55.45%; H, 7.30%; N, 11.05%.

Chromatography analysis (t.l.c. and HPLC) shows no presence of impurities, and the $^{1}H$ n.m.r. spectrum confirms the molecular structure.

EXAMPLE 4

Synthesis of benzyloxycarbonyl-phenylalanyl-gem-diamino-phenylalanine hydrochloride. Z-Phe-g Phe-H.HCl

1.2 equivalents of BTI are dissolved in a 1:1 (v/v) water-dimethylformamide mixture (or in a 1:1 (v/v) mixture of 1% trifluoroacetic acid in water with dimethylformamide). 1 equivalent of Z-Phe-Phe-$NH_2$ dissolved in dimethylformamide is added to this solution. During the reaction (about six hours) an inert gas is bubbled through in order to facilitate removal of the carbon dioxide which forms. Having verified the disappearance of Z-Phe-Phe-$NH_2$, the reaction is suspended by evaporating the solvent mixture, the oily residue is washed several times with ethyl ether, dried in a vacuum oven over $P_2O_5$, and again dissolved in methanol. The required product was precipitated by adding a stoichiometric quantity of HCl dissolved in water. After repeated washing with ethyl ether, the final product was again dried under vacuum over $P_2O_5$ and collected. M.P. = 147°C (dec.).

$[\alpha]_{20}^{546}$ = 65.2 (C = 1 in dimethylformamide).

Elementary analysis for $C_{25}H_{28}N_3O_3Cl$:

Theoretical: C, 66.16%; H, 6.17%; N, 9.26%.

Found: C, 65.80%; H, 6.09%; N, 9.14%.

Chromatography analysis (t.l.c. and HPLC) shows no presence of impurities, and the $^1H$ n.m.r. spectrum confirms the molecular structure.

EXAMPLE 5

Synthesis of tert-butyloxycarbonyl-alanyl-gem-diamino-phenylalanyl-D-phenylalanine-benzyloxycarbonyl. Boc-Ala-g Phe-D Phe-Z.

1.1 equivalents of Z-D Phe are dissolved in dimethylformamide, the solution is cooled with an ice bath, and 1.5 equivalents of N-hydroxybenzotriazole dissolved in dimethylformamide and 1.1 equivalents of dicyclohexylcarbodiimide dissolved in tetrahydrofuran are added to the solution. After 20 minutes, 1.0 equivalents of Boc-Ala-g Phe-H.HCl and 1.1 equivalents of N-methylmorpholine are added to the mixture. The ice bath is removed after about one hour. Having verified the disappearance of Boc-Ala-g Phe-H.HCl after four hours, the reaction mixture is filtered, the residue is washed with tetrahydrofuran and discarded. The filtrate solution and the wash waters are combined, evaporated to a volume of about 10 ml and treated with an excess of water. A white caseous precipitate is obtained, which is filtered and washed abundantly with portions of a 5% citric acid solution, a 5% sodium bicarbonate solution and water.

The resultant product is dissolved in tetrahydrofuran and crystallised by adding 30-50$^{\circ}$ petroleum ether. M.P. = 195-197$^{\circ}$C.

$[\alpha]_{20}^{546}$ = 9.96 (C = 1.5 in dimethylformamide).

Elementary analysis for $C_{33}H_{40}N_4O_6$:

Theoretical: C, 67.35%; H, 6.80%; N, 9.52%.

Found: C, 67.50%; H, 6.92%; N, 9.38%.

Chromatography analysis (t.l.c. and HPLC) shows no presence of impurities, and the $^{1}H$ n.m.r. spectrum confirms the molecular structure.

EXAMPLE 6

Synthesis of tert-butyloxycarbonylphenylalanyl-gem-diamino-L-phenylalanyl-m-glycyl-leucylmethionine amide. Boc-Phe-g Phe-m Gly-Leu-Met-$NH_2$.

1 equivalent of HOOC-$CH_2$-CO-Leu-Met-$NH_2$ (HO-m Gly-Leu-Met-$NH_2$) is dissolved in tetrahydrofuran and the solution is cooled with an ice bath, after which 1.5 equivalents of N-hydroxybenzotriazole dissolved in dimethylformamide and 1.1 equivalents of dicyclohexylcarbodiimide dissolved in tetrahydrofuran are added.

After 20 minutes, 1.0 equivalent of Boc-Phe- g Phe-H.HCl and 1.1 equivalents of N-methylmorpholine are added to the cold mixture. The ice bath is removed after about one hour, and the mixture is left to react overnight at ambient temperature. After filtering off the precipitate, which is washed with tetrahydrofuran, the combined solution and wash liquors are reduced to about 10 ml and a white flaky precipitate is obtained by subsequent treatment with an excess of water. The precipitate is filtered off and washed with numerous portions of 5% citric acid solution, 5% sodium bicarbonate and water. After drying over $P_2O_5$ under vacuum, the solid residue is further washed with ethyl ether, dried and collected. M.P. = 242-245°C.

$[\alpha]^{546}_{20}$ = 12.33 (C = 0.74 in dimethylformamide).

Elementary analysis for $C_{36}H_{52}N_6O_7S$:

Theoretical: C, 60.67%; H, 7.30%; N, 11.80%.

Found: C, 60.35%; H, 7.09%; N, 11.69%.

Chromatography analysis (t.l.c. and HPLC) shows no presence of impurities, and the $^1H$ n.m.r. spectrum confirms the molecular structure.

0084691

CASE 1463

CLAIM:

1. A method for preparing N-monoacylated gem-diamino derivatives of formula

$$P-(A)_n-NH-\underset{\underset{R}{|}}{CH}-NH_3^+X^-$$

where P is an acyl residue; A is a residue of a natural or synthetic aminoacid or hydroxyacid, R is hydrogen, or an alkyl, phenyl, phenylalkylene, hydroxyphenylalkylene, hydroxyalkylene, aminoalkylene, acylaminoalkylene, guanidinylalkylene, imidazolylalkylene, indolylalkylene, mercaptoalkylene, alkylmercaptoalkylene or carboxyalkylene group or one of their derivatives; and n is 0, 1 or greater than 1, in which case the sequence $(A)_n$ can be constituted by residues which are equal or different to each other, consisting of reacting amides of formula

$$P-(A)_n-NH-\underset{\underset{R}{|}}{CH}-C$$

with I,I-bis(trifluoroacetoxy)iodobenzene.

European Patent Office

# EUROPEAN SEARCH REPORT

0084691

Application number

EP 82 20 1611

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| D,Y | JOURNAL OF ORGANIC CHEMISTRY, vol. 44, no. 10, 11 May 1979 RADHAKRISHNA et al. "New method for direct conversion of amides to amines" pages 1746, 1747 * Page 1746, column 2, lines 14-16 * | 1 | C 07 C 103/52 |
| | --- | | |
| Y | JOURNAL OF THE AMERICAN CHEMICAL SOCIETY, vol. 99, no. 24, 23 November 1977 CHOREV et al. "A general approach to retro-isomeric linear peptide synthesis" pages 8075, 8076 * Complete * | 1 | |
| | ----- | | |
| | | | TECHNICAL FIELDS SEARCHED (Int. Cl. 3) |
| | | | C 07 C 103/52 |

The present search report has been drawn up for all claims

| Place of search | Date of completion of the search | Examiner |
|---|---|---|
| BERLIN | 17-03-1983 | BREW C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document